# EUROPEAN PATENT SPECIFICATION

(11) **EP 1 693 007 B1**
(45) Date of publication and mention of the grant of the patent: **09.10.2013**
(21) Application number: 06002440.3
(22) Date of filing: 07.02.2006
(51) Int. Cl.: A61B 17/064

(54) **Surgical staple**
Chirurgische Klammer
Agrafe chirurgicale

(30) Priority: 17.02.2005 US 59922
(43) Date of publication of application: 23.08.2006
(73) Proprietor: Covidien LP, Mansfield, MA 02048 (US)
(72) Inventor: Emmons, Clifford, Oakville CT 06779 (US)
(74) Representative: HOFFMANN EITLE

(56) References cited:
- EP-A- 0 509 815
- EP-A- 0 852 128
- WO-A-2004/105621
- WO-A2-2004/060169
- JP-A- 7 124 166
- US-A1- 2002 133 181
- US-A1- 2002 188 318
- US-A1- 2003 014 064
- US-A1- 2004 028 502

## Description

### BACKGROUND

### 1. Technical Field

The present disclosure relates to a surgical staple.

### 2. Background of Related Art

Surgical stapling apparatus are widely used in surgical procedures to fasten body tissue in a quick and efficient manner by driving fasteners or staples into the body tissue. In certain types of staplers, a single staple is typically formed against an anvil, e.g., skin staplers, for approximating body tissue.

In other types of staplers, such as those for mechanically stitching together hollow organs, a main linear drive (i.e., a cam member) moves longitudinally in a direction transverse to the direction that the staples are to be driven. Typically, such staplers employ a number of staple drive members or pusher elements which pass through grooved slots of a staple retaining cartridge, such slots being arranged end-to-end in rows. Under normal operation, the longitudinally moving cam member passes into and through the staple retaining cartridge, contacting cam surfaces on the drive members as the cam member passes through the grooved slots. Thus, the drive members convert linear motion from the cam member to transverse staple motion, thereby driving the row(s) of staples into the body tissue to be fastened.

An example of such an arrangement is described in commonly assigned U.S. Pat. No. 3,490,675 to Green et al.. U.S. Pat. No. 3,490,675 generally discloses a drive member having a pusher plate, guide rails and a V-shaped portion for contacting the drive cam. A further modification is illustrated in commonly assigned U.S. Pat. No. 3,499,591 to Green. U.S. Pat. No. 3,499,591 discloses a two staple driver capable of driving double rows of staples in a single cam stroke for greater holding strength than a single row of staples.

Typically, as the surgical stapler is being fired (i.e., the staple driver is being advanced distally to expel the staples from a staple cartridge, through the body tissue and deform against a staple anvil) there is a flow of the body tissue, grasped between the staple cartridge and the staple anvil, in a distal direction.

Each of the above-described staple systems are commonly employed to drive conventional U-shaped staples having two opposed equal length legs terminating at one end in a sharpened skin or tissue-piercing point and connected at an opposite end by a linear bridge. When deformed, such staples tend to form a B-shape, wherein the legs are in a position to re-puncture the tissue being sutured. Typically, the tissue piercing points of the staples are oriented outwardly, i.e., the distal-most tip of each point is located along the inner surface of each leg and tapers outwardly therefrom to the outer surface of each leg. Further, since the legs of such staples have an equal length, as the stapler is fired, each leg of such staples will exit the staple pocket and will simultaneously meet the distal flow of body tissue. As is evident, the body tissue will continue to flow, in the distal direction, when passing the proximal-most leg of the staple due to the angled orientation of the tissue piercing point. Further, it is evident that the proximal most leg of each staple is unsupported along an inner surface thereof while the distal most leg of each staple is supported along the distal-most surface thereof by the distal wall of each staple pocket. Thus, as the staples are expelled from the staple pockets formed in the staple cartridge the distal flow of body tissue will cause the proximal-most leg of the staple to deflect in a distal direction.

Accordingly, it can be appreciated that there exists a continuing need for a new and improved surgical staple which reduces the amount of distal flow of body tissue that occurs, between the staple cartridge and the staple anvil, during a surgical stapling operation utilizing a surgical stipler.

EP 0 852 128 Al describes a surgical clip for connecting at least two bio-organic tissue members together, the clip comprising a body having two spaced legs and formed into a U-shape. Each leg has a distal end which can be inserted into an opening in bone and each has a proximal end. The proximal ends of said at least two legs are connected to corresponding ends of a deformable bridge comprising two spaced apart bridge sections with a gap disposed between the bridge sections.

US 2002/0133181 describes a four-spikes surgical skin staple which is composed of two stems which form a 60 degree angle V-shaped figure and four spikes, which are composed of two identical outer long spikes and two identical inner short spikes. Each of the two identical outer long spikes is fixed at a 60 degree angle to the outer end of each stem and each of the two identical inner short spikes is fixed at a 60 degree angle to the middle of each stem. The two outer long spikes are used to approximate the surgical incision wound and the two inner short spikes are used to stabilize the incision skin edges to prevent the overlapping of the skin edges.

WO 2004/105621 describes a surgical fastener for use with an anastomosis of two tissues which includes a base leg and an upright leg. The base leg is selectively deformable and includes a traumatic tip for piecing tissue. The surgical fastener also includes at least one capillary disposed on the base leg which has a reservoir defined therein for retaining a liquid, e.g. bioadhesive, bonding agent, medicament, etc. Each of the capillaries is ruptureable upon deformation of the surgical fastener to dispense the liquid to the anastomosis site.

US 2004/0028502 describes a generally U-shaped surgical staple which comprises a base and a pair of generally "L"-shaped legs extending substantially perpendicularly from opposite ends of the base respectively. The legs in use of the staple are bent through approximately 90° relative to the base. To effect a greater compression of the stapled tissue the legs include a penetration portion adjacent the tip and a compressive structure which, due to its increased height relative to that of the penetrative portion, spreads the compressive forces of the staple further along the length of the incision being closed. The compressive portion also provides a depth stop to avoid the tip penetrating too deeply into the tissue in which it is deployed.

US 2003/0014064 describes an apparatus, systems and methods for providing an effective tool for intraluminally directed vascular anastomosis of a graft vessel to a receiving blood vessel that is performed according to a minimally invasive procedure.

EP 0 509 815 describes a surgical staple which is used in joining the skin or fascia of a patient, and is especially desired to be used with mesh placed over a cut organ, such as the kidney. The staple is adapted to be formed about a central anvil and former. The configuration of the staple is such that at initial contact the points of the staple are formed at acute angles to the central portion of the staple. In this fashion, upon forming, a modified "B" shape is derived.

FR 2 743 490 describes a staple having two uneven legs and a bridge therebetween and made of titanium or a material having similar characteristics.

JP 7-124166 describes a staple having two uneven legs and a bridge therebetween. In addition a staple storage cartridge is shown loaded with staples.

### SUMMARY

The subject application is directed to a unique surgical staple which includes a first leg having a distal end portion and a proximal end, wherein the first leg has a first predetermined length, a second leg having a distal end portion and a proximal end, wherein the second leg has a second predetermined length which is less than the first predetermined length of the first leg and a bridge member interconnecting the proximal ends of the first and second legs.

In one preferred embodiment the distal end portion of the first leg terminates in a distal angled surface while the distal end portion of the second leg terminates in a distal angled surface. Preferably, the distal angled surface of the first leg and the distal angled surface of the second leg are oriented in the same direction as one another and more preferably, the distal angled surface of the second leg is oriented toward the bridge member. It is envisioned that the distal angled surface of the first leg can be oriented toward the bridge member.

The first leg and the second leg each define a respective longitudinal axis. Preferably, the distal angled surface of the first leg is angled from about 45° to about 55° relative to the longitudinal axis thereof and the distal angled surface of the second leg is angled from about 45° to about 55° relative to the longitudinal axis thereof.

In one embodiment, the surgical staple includes a bridge member defining a first distance and the distal angled surface of the first leg is spaced from the distal angled surface of the second leg a second distance. Preferably, the second distance is greater than the first distance.

The surgical staple includes a bridge member defining an axis. Accordingly, the longitudinal axis of the first leg is angled an amount which is less than about 90° relative to the axis defined by the bridge member and the longitudinal axis of the second leg is angled an amount which is less than about 90° relative to the axis defines by the bridge member.

The first leg and the bridge member define a first plane and the second leg and the bridge member define a second plane. In accordance with the present disclosure, it is contemplated that the second plane is angled an amount, about the axis defined by the bridge member, with respect to the first plane.

In accordance with an alternative embodiment of the present disclosure, the surgical staple includes a first leg having a distal end portion and a proximal end, wherein the distal end portion of the second leg terminates in an angled distal surface, a second leg having a distal end portion and a proximal end, wherein the distal end portion of the second leg terminates in an angled distal surface and a bridge member interconnecting the proximal ends of the first and second legs, wherein the distal angled surface of the first leg and the distal angled surface of the second leg are oriented in the same direction as one another.

These and other features of the surgical staple will become more readily apparent to those skilled in the art from the following detailed description of the subject application, the accompanying drawings and the claims.

The invention is defined in the claims.

### BRIEF DESCRIPTION OF THE DRAWINGS

The accompanying drawings, which are incorporated in and constitute a part of this specification, illustrate exemplary embodiments of the disclosure and, together with the general description given above, and the detailed description of the embodiments given below, serve to explain the principles of the present disclosure.
FIG. 1 is a front elevational view of a surgical staple in accordance with the principles of the present disclosure;
FIG. 2 is a side elevational view of the staple of FIG. 1;
FIG. 3 is an enlarged view of a distal end of a leg, indicated by area "3, of the staple shown in FIG. 1;
FIG. 4 is a cross-sectional schematic side elevational view of prior art staple being expelled from a distal end portion of a conventional surgical stapling apparatus;
FIG. 5 is an enlarged view of a prior art surgical staple being expelled from a conventional surgical stapling apparatus, as indicated by area "5 of FIG. 4;
FIG. 6 is a cross-sectional side elevational view of the surgical staple of FIG. 1 being expelled from a distal end portion of a conventional surgical stapling apparatus; and
FIG. 7 is an enlarged view of a surgical staple, in accordance with the principles of the present disclosure, being expelled from a conventional surgical stapling apparatus, as indicated by area "7 of FIG. 5.

### DETAILED DESCRIPTION OF PREFERRED EMBODIMENTS

Preferred embodiments of the presently disclosed surgical stapler will now be described in detail with reference to the drawing figures wherein like reference numerals identify similar or identical elements. In the drawings, and in the description which follows, the term "proximal", as is traditional, will refer to the end of the device or element which is closest to the operator, while the term "distal" will refer to the end of the device or element which is furthest from the operator.

It is envisioned that the surgical staples disclosed herein can be used with several known types of surgical staplers for various procedures (examples include: end-to-end anastomosis, "EEA^{™}"; circular end-to-end anastomosis, "CEEA^{™}"; gastrointestinal anastomosis, "GIA^{™}"; endoscopic gastrointestinal anastomosis, "Endo GIA^{™}"; and transverse anastomosis, "TA^{™}" staplers available from United States Surgical, a division of Tyco Health-Care Group, LP, Norwalk, CT.) with each stapler including a staple anvil which is adjustably approximated relative a staple cartridge.

A typical staple cartridge generally has at least two laterally spaced rows of staple pockets having a staple disposed therein, while the staple anvil generally includes staple deforming depressions formed therein which staple deforming depressions are aligned with the rows of staple pockets in the cartridge. In use, each of the surgical staplers involves the gripping of body tissue to be fastened, the ejecting of individual staples, the forcing of staples through the gripped body tissue and the closing of the staples against the staple deforming depressions formed in the anvil of the stapler. While the following description will generally relate to a gastrointestinal anastomosis surgical stapler, it will be understood that the description of the surgical staple in accordance with the principles of the present disclosure can be applied to any of the aforementioned surgical staplers.

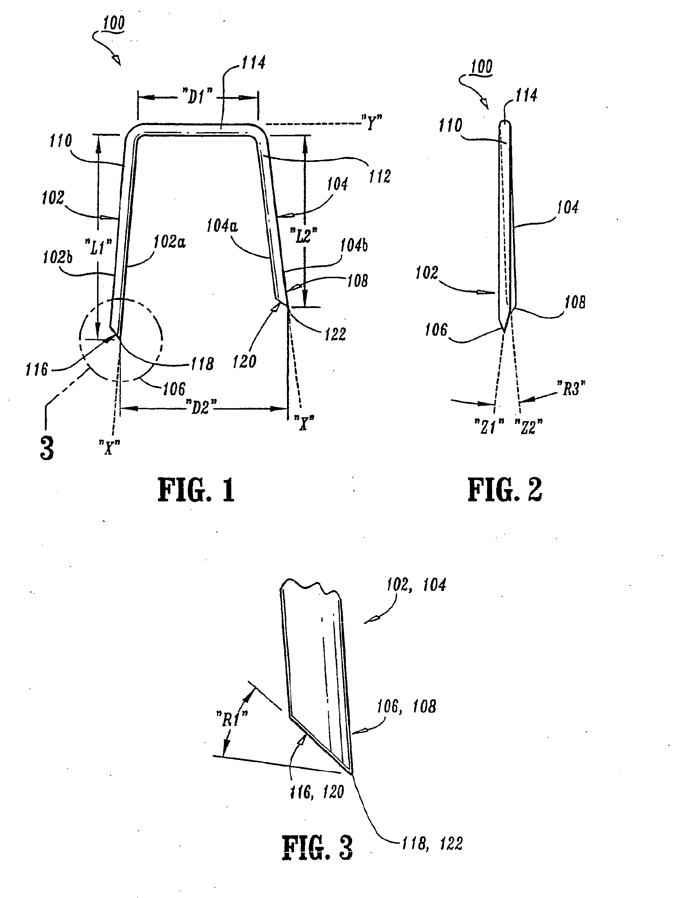

Referring now to FIGS. 1-3, a surgical staple, in accordance with the principles of the present disclosure, is shown generally as reference numeral 100. Staple 100 includes a first and a second leg 102 and 104, respectively, each terminating in a beveled distal end portion 106, 108, and interconnected at a proximal end 110, 112 thereof by a bridge portion 114. As shown in FIG. 1, first leg 102 has a length "L1" while second leg 104 has a length "L2", wherein length "L1" is greater then length "L2".

Preferably, in accordance with the present disclosure, beveled distal end portion 106 of first leg 102 is defined by a distal angled surface 116 terminating in a sharpened distal tip 118 while beveled distal end portion 108 of second leg 104 is defined by a distal angled surface 120 terminating in a sharpened distal tip 122. Preferably, distal angled surfaces 116 and 120 are oriented in the same direction. More preferably, distal angled surface 116 of first leg 102 is oriented in a direction away from bridge portion 114 while distal angled surface 120 of second leg 104 is oriented in a direction toward bridge portion 114.

Stated differently, first leg 102 defines an inner surface 102a oriented toward second leg 104 and an outer surface 102b defined by the side opposite inner surface 102a, while second leg 104 defines an inner surface 104a oriented toward first leg 102 and an outer surface 104b defined by the side opposite inner surface 104a. As such, in accordance with the present disclosure, distal angled surface 116 of first leg 102 is oriented toward outer surface 102b while distal angled surface 120 of second leg 104 is oriented toward inner surface 104a. Preferably, distal angled surface 120 of second leg 104 is angled in a direction opposite to distal angled surface 116 of first leg 102.

As shown in FIG. 1 and in greater detail in FIG. 3, distal angled surfaces 116, 120 are angled an amount "R1" relative to a longitudinal axis of each leg 102, 104. Preferably, distal angled surfaces 116, 120 are angled an amount "R1" ranging from about 45° to about 55° relative to the longitudinal axis. While an angular amount "R1" ranging from about 45° to about 55° relative to the longitudinal axis is preferred, it is envisioned that different angular orientations of distal angled surfaces 116, 120 are possible.

While it is preferred that distal angled surfaces 116, 120 are oriented in the same direction, it is envisioned that while distal angled surface 120 of second leg 104 is oriented toward bridge portion 114, distal angled surface 116 of first leg 102 can be oriented in any number of directions including oriented toward bridge portion 114.

Returning to FIG. 1, it is seen that first leg 102 and second leg 104 diverge from one another. In other words, proximal ends 110, 112 are spaced a distance "D1" from one another while distal end portions 106, 108 are spaced a distance "D2" from one another, wherein distance "D2" is greater than distance "D1". Preferably, distance "D1" ranges from about 0.295 to about 0.302 cm (about 0.116 to about 0.119 inches) while distance "D2" ranges from about 0.368 to about 0.419 cm (about 0.145 to about 0.165 inches). Stated differently, bridge portion 114 defines a bridge axis "Y" while first and second legs 102, 104 each define a longitudinal axis "X" wherein longitudinal axes "X" are angled an amount "R2" relative to an axis which is orthogonal to the "Y" axis. Accordingly, the longitudinal "X" axis of each leg 102, 104 is angled an amount "R2" which is less than about 90°.

Referring now to FIG. 2, is it seen that first leg 102 and bridge 114 define a first plane "Z1" while second leg 104 and bridge portion 114 define a second plane "Z2", angled an amount "R3" relative to one another. Preferably, first plane "Z1" and second plane "Z2" are angled an amount "R3" which offsets distal end portion 106 and distal end portion 108 from one another by an amount equal to about 0.015 cm (0.006 inches) in a direction orthogonal to first plane "Z1". In this manner, as first and second legs 102, 104 are deformed during a staple forming process, distal end portions 106, 108 will be out of plane with respect to one another, thus not interfering with one another.

It is envisioned that surgical staple 100 may be formed from a medical grade material wire, such as for example, stainless steel, titanium, or other similar material suitable for surgical utilization. Preferably, surgical staple has a cross-section which is substantially circular. Alternatively, surgical staple 100 can be formed from a wire having a substantially oval, rectangular, semi-circular or other cross-sectional configuration which can be utilized to construct staples.

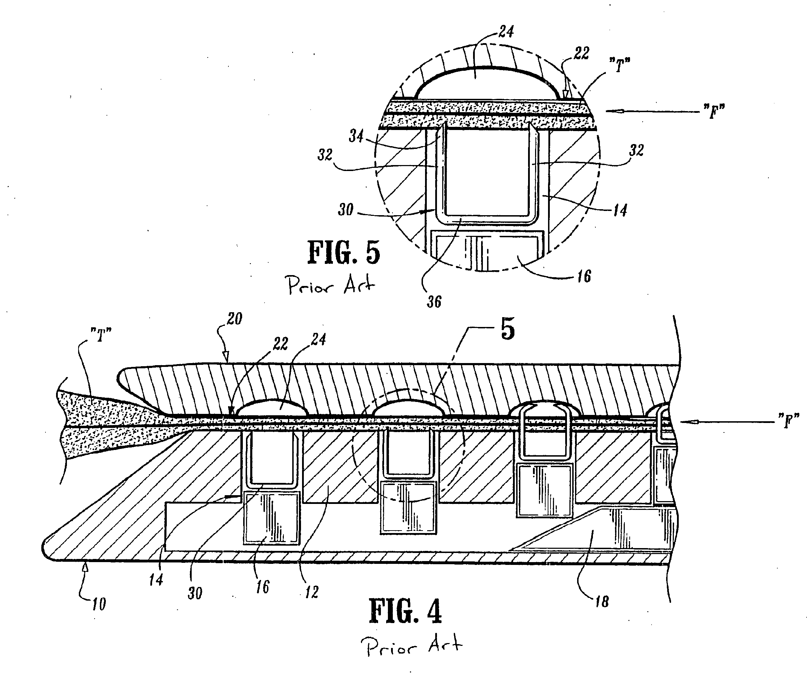

Turning now to FIGS. 4 and 5, a cross-sectional schematic side elevational view of prior art surgical staples being expelled from a distal portion of a conventional surgical stapling apparatus is shown. As seen in FIG. 4, the distal portion of the conventional surgical stapling apparatus includes a first jaw 10 defining a staple cartridge 12 having a plurality of elongated slots 14 for storing conventional staples 30 therein and a staple drive member 18 slidably extending therethrough. Drive member 18 being configured and adapted to transform a linear movement thereof into a transverse movement of a pusher 16 disposed in each elongated slot for transversely expelling conventional staples 30 therefrom. The distal portion of the conventional surgical stapling apparatus further includes a second jaw 20 having an anvil 22 provided on the inner surface thereof and includes a plurality of anvil pockets 24, aligned with slots 14, configured and adapted to deform the legs of staples 30. First and second jaws 10, 20 are pivotably mounted to one another in order to clamp body tissue "T" between staple cartridge 12 and anvil 22.

Conventional staples 30 include a pair of opposed legs 32 terminating at one end in a sharpened distal tip 34 and connected at an opposite end by a bridge member 36. As seen in FIGS. 4 and 5, each leg 32 of conventional staples 30 has a length which is equal to one another. Moreover, legs 32 of conventional staples 30 are parallel to one another and are substantially orthogonal to bridge member 36.

In use, with body tissue "T" clamped between staple cartridge 12 and anvil 22, as drive member 18 is advanced distally through staple cartridge 12, drive member 18 forces each pusher 16 in a transverse direction through slots 14 and ultimately staples 30 out of slots 14 and into body tissue "T". As staples 30 are expelled and formed, from a proximal end of the stapling apparatus toward a distal end of the stapling apparatus, there is a flow "F" of body tissue "T" in a distal direction. Accordingly, since legs 32 of each staple 30 have an equal length they are simultaneously expelled from slots 14. As seen in detail in FIG. 5, when distal tip 34 of legs 32 meet distal flow "F" of body tissue "T", the distal most leg remains upright, since the distal most leg is supported along a distal side thereof (i.e., supported by the inner wall of slot 14), while the proximal most leg tends to deflect in a distal direction since the proximal most leg is not supported along a distal side thereof. Moreover, as seen in FIG. 5, since distal tips 34 are angled outwardly (i.e., the longer portion of the legs is located inside of the shorter portion) body tissue "T" will tend to ride up and over the angled surface of the proximal most leg thus permitting the continued flow "F" of body tissue "T".

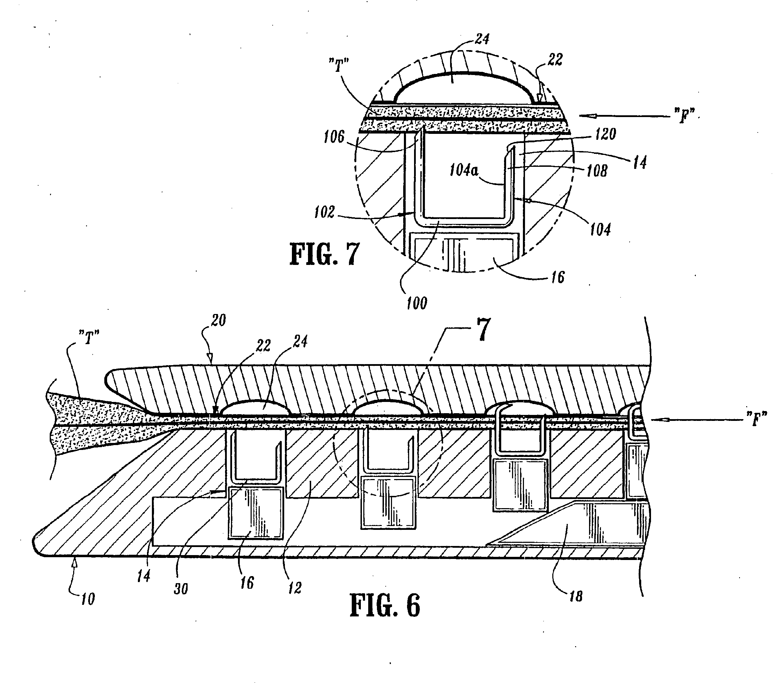

Turning now to FIGS. 6 and 7, a cross-sectional schematic side elevational view of surgical staples 100, in accordance with the principles of the present disclosure, are being expelled from a distal portion of a conventional surgical stapling apparatus, as described above, is shown. As seen in FIG. 4, surgical staples 100 are deliberately placed within slots 14 of staple cartridge 12 such that first leg 102 is positioned distally of second leg 104. In particular, surgical staples 100 are placed within slots 14 such that the longer leg (i.e., leg 102) is located in the distal portion slot 14 while the shorter leg (i.e., leg 104) is located in the proximal portion of slot 14.

In use, as seen in FIG. 6 and in greater detail in FIG. 7, with body tissue "T" clamped between staple cartridge 12 and anvil 22, as drive member 18 is advanced distally through staple cartridge 12, drive member 18 forces each pusher 16 in a transverse direction through slots 14 and ultimately staples 100 out of slots 14 and into body tissue "T". Since staples 100 have uneven legs 102, 104 and since longer leg 102 is located distally of shorter leg 104, as staples 100 are expelled from slots 14, longer legs 102 will project out of staple cartridge 12 before shorter legs 104. As such, distal end portion 106 of legs 102 will penetrate body tissue "T" before distal end portion 108 of legs 104. Moreover, distal end portion 106 of legs 102 will meet distal flow "F' of body tissue "T" before distal end portion 108 of legs 104.

As seen in detail in FIG. 7, when distal end portion 106 of legs 102 meet distal flow "F" of body tissue "T", legs 102 remain upright since they are supported along outer surface 102B thereof by the inner wall of slot 14. In addition, legs 102 interfere with and slow down the rate of distal flow "F" of body tissue "T" in effect causing body tissue "T" to "back-up" so that when distal end portion 108 of legs 104 penetrate body tissue "T" they will not be deflected in a distal direction when passing through body tissue "T".

Moreover, as seen in FIG. 7, since distal angled surface 120 of second leg 104 of staples 100 are preferably angled to face inner surface 104a of second leg 104, the effect of body tissue "T" riding up and over distal end portion 108 of second leg 104 is reduced in present staple 100 as compared to conventional staples 30.

It will be understood that various modifications may be made to the embodiments disclosed herein. Therefore, the above description should not be construed as limiting, but merely as exemplifications of preferred embodiments. For example, the subject invention, which has been described with respect to a surgical apparatus wherein the staples are fired transverse to the longitudinal axis, may alternatively be described with respect to an apparatus wherein the staples are driven longitudinally against an anvil having a staple forming surface extending transversely and disposed opposite the staples. Those skilled in the art will envision other modifications within the scope of the claims appended hereto.

## Claims

1. An assembly of a staple cartridge (12)and surgical staples (100), each surgical staple comprising:
a first leg (100) having a distal end portion (106) and a proximal end (110), wherein the first leg has a first predetermined length;
a second leg (104) having a distal end (108) portion and a proximal end (112), wherein the second leg has a second predetermined length which is less than the first predetermined length of the first leg; and
a bridge member (114) interconnecting the proximal ends of the first and second legs,
the staple cartridge comprising slots (14) within each of which one of the surgical staples is placed such that in use the first leg will project out of the staple cartridge before the second leg will project out of the staple cartridge; the assembly being **characterised in that** the first leg and the bridge member define a first plane and the second leg and the bridge member define a second plane which is angled with respect to the first plane about the axis defined by the bridge member.

2. The assembly according to claim 1, wherein the distal end portion of the first leg terminates in a distal angled surface (116) and wherein the distal end portion of the second leg terminates in a distal angled surface (120).

3. The assembly according to claim 2, wherein the distal angled surface of the first leg and the distal angled surface of the second leg are oriented in the same direction.

4. The assembly according to claim 2 or 3, wherein the distal angled surface of the second leg is oriented toward the bridge member.

5. The assembly according to claim 4, wherein the first leg and the second leg each define a respective longitudinal axis, wherein the distal angled surface of the first leg is angled from about 45° to about 55° relative to the longitudinal axis thereof and wherein the distal angled surface of he second leg is angled from about 45° to about 55° relative to the longitudinal axis thereof.

6. The assembly according to claim 5, wherein the bridge member defines a distance and wherein the distal angled surface of the first leg is spaced a distance from the distal angled surface of the second leg, wherein the distance between the distal angled surfaces of the first and second legs is greater than the distance defined by the bridge member.

7. The assembly according to claim 5, wherein the bridge member defines an axis and wherein the longitudinal axis of the first leg is angled an amount which is less than about 90° relative to the axis defined by the bridge member and wherein the longitudinal axis of the second leg is angled an amount which is less than about 90° relative to the axis defined by the bridge member.

## Patentansprüche

1. Aufbau einer Klammerkassette (12) und chirurgischer Klammern (100), jede chirurgische Klammer mit:
einem ersten Bein (100), das einen distalen Endabschnitt (106) und ein proximales Ende (110) aufweist, wobei das erste Bein eine erste festgelegte Länge aufweist;
einem zweiten Bein (104), das einen distalen Endabschnitt (108) und ein proximales Ende (112) aufweist, wobei das zweite Bein eine zweite festgelegte Länge aufweist, die geringer ist als die erste festgelegte Länge des ersten Beins; und
einem Brückenelement (114), das die proximalen Enden des ersten und zweiten Beins miteinander verbindet,
wobei die Klammerkassette Spalte (14) aufweist, in denen jeweils eine der chirurgischen Klammern platziert ist, sodass das erste Bein bei Verwendung aus der Klammerkassette hervorsteht bevor das zweite Bein aus der Klammerkassette hervorsteht; und
wobei der Aufbau **dadurch gekennzeichnet ist, dass** das erste Bein und das Brückenelement eine erste Ebene definieren und das zweite Bein und das Brückenelement eine zweite Ebene definieren, die im Verhältnis zu der ersten Ebene um die durch das Brückenelement definierte Achse abgewinkelt ist.

2. Aufbau nach Anspruch 1, bei dem der distale Endabschnitt des ersten Beins in einer distalen abgewinkelten Fläche (116) endet und bei dem der distale Endabschnitt des zweiten Beins in einer distalen abgewinkelten Fläche (120) endet.

3. Aufbau nach Anspruch 2, bei dem die distale abgewinkelte Fläche des ersten Beins und die distale abgewinkelte Fläche des zweiten Beins in der gleichen Richtung ausgerichtet sind.

4. Aufbau nach Anspruch 2 oder 3, bei dem die distale abgewinkelte Fläche des zweiten Beins in Richtung des Brückenelements ausgerichtet ist.

5. Aufbau nach Anspruch 4, bei dem das erste Bein und das zweite Bein jeweils eine jeweilige Längsachse definieren, wobei die distale abgewinkelte Fläche des ersten Beins relativ zu seiner Längsachse mit in etwa 45° bis in etwa 55° abgewinkelt ist und wobei die distale abgewinkelte Fläche des zweiten Beins relativ zu seiner Längsachse mit in etwa 45° bis in etwa 55° abgewinkelt ist.

6. Aufbau nach Anspruch 5, bei dem das Brückenelement einen Abstand definiert und bei dem die distale abgewinkelte Fläche des ersten Beins mit einem Abstand von der distalen abgewinkelten Fläche des zweiten Beins beabstandet ist, wobei der Abstand zwischen den distalen abgewinkelten Flächen des ersten und zweiten Beins größer ist als der durch das Brückenelement definierte Abstand.

7. Aufbau nach Anspruch 5, bei dem das Brückenelement eine Achse definiert und bei dem die Längsachse des ersten Beins um einen Betrag abgewinkelt ist, der geringer als in etwa 90° relativ zu der durch das Brückenelement definierten Achse ist und bei dem die Längsachse des zweiten Beins um einen Betrag abgewinkelt ist, der geringer als in etwa 90° relativ zu der durch das Brückenelement definierten Achse ist.

## Revendications

1. Ensemble composé d'une cartouche d'agrafes (12) et d'agrafes chirurgicales (100), chaque agrafe chirurgicale comprenant :
une première patte (100) ayant une partie d'extrémité distale (106) et une extrémité proximale (110), dans lequel la première patte a une première longueur prédéterminée ;
une seconde patte (104) ayant une partie d'extrémité distale (108) et une extrémité proximale (112), dans lequel la seconde patte a une seconde longueur prédéterminée qui est inférieure à la première longueur prédéterminée de la première patte ; et
un élément de pont (114) interconnectant les extrémités proximales des première et seconde pattes,
la cartouche d'agrafes comprenant des fentes (14) à l'intérieur de chacune desquelles l'une des agrafes chirurgicales est placée de sorte que, à l'usage, la première patte fait saillie de la cartouche d'agrafes avant que la seconde patte ne fasse saillie de la cartouche d'agrafes ; l'ensemble étant **caractérisé en ce que** la première patte et l'élément de pont définissent un premier plan et **en ce que** la seconde patte et l'élément de pont définissent un second plan qui est coudé par rapport au premier plan autour de l'axe défini par l'élément de pont.

2. Ensemble selon la revendication 1, dans lequel la partie d'extrémité distale de la première patte se termine par une surface coudée distale (116) et dans lequel la partie d'extrémité distale de la seconde patte se termine par une surface coudée distale (120).

3. Ensemble selon la revendication 2, dans lequel la surface coudée distale de la première patte et la surface coudée distale de la seconde patte sont orientées dans la même direction.

4. Ensemble selon la revendication 2 ou 3, dans lequel la surface coudée distale de la seconde patte est orientée vers l'élément de pont.

5. Ensemble selon la revendication 4, dans lequel la première patte et la seconde patte définissent chacune un axe longitudinal respectif, dans lequel la surface coudée distale de la première patte est coudée d'environ 45° à environ 55° par rapport à son axe longitudinal et dans lequel la surface coudée distale de la seconde patte est coudée d'environ 45° à environ 55° par rapport à son axe longitudinal.

6. Ensemble selon la revendication 5, dans lequel l'élément de pont définit une distance et dans lequel la surface coudée distale de la première patte est espacée selon une distance par rapport à la surface coudée distale de la seconde patte, dans lequel la distance entre les surfaces coudées distales des première et seconde pattes est supérieure à la distance définie par l'élément de pont.

7. Ensemble selon la revendication 5, dans lequel l'élément de pont définit un axe et dans lequel l'axe longitudinal de la première patte est coudé selon une quantité qui est inférieure à environ 90° par rapport à l'axe défini par l'élément de pont et dans lequel l'axe longitudinal de la seconde patte est coudé selon une quantité qui est inférieure à environ 90° par rapport à l'axe défini par l'élément de pont.
